(19) Europäisches Patentamt / European Patent Office / Office européen des brevets

(11) **EP 4 123 656 A1**

(12) **EUROPEAN PATENT APPLICATION**

(43) Date of publication:
   **25.01.2023  Bulletin 2023/04**

(21) Application number: **21187053.0**

(22) Date of filing: **21.07.2021**

(51) International Patent Classification (IPC):
   **G16H 10/60** (2018.01)

(52) Cooperative Patent Classification (CPC):
   **G16H 10/60**

(84) Designated Contracting States:
   **AL AT BE BG CH CY CZ DE DK EE ES FI FR GB GR HR HU IE IS IT LI LT LU LV MC MK MT NL NO PL PT RO RS SE SI SK SM TR**
   Designated Extension States:
   **BA ME**
   Designated Validation States:
   **KH MA MD TN**

(71) Applicant: **Koninklijke Philips N.V.**
   **5656 AG Eindhoven (NL)**

(72) Inventor: **VAN BERKEL, Joep Joseph Benjamin Nathan**
   **5656 AE Eindhoven (NL)**

(74) Representative: **Philips Intellectual Property & Standards**
   **High Tech Campus 52**
   **5656 AG Eindhoven (NL)**

(54) **ESTIMATING RELEVANCE OF PARTS OF MEDICAL RECORDS**

(57)   A mechanism for estimating the relevance or importance of sections of unstructured (medical) textual data. Different sections are visually emphasized responsive to a user input, and one or more characteristics of when the sections are visually emphasized are monitored. Other characteristics of the sections may also be monitored or determined. These characteristics are then processed to predict or determine a relevance of each section.

FIG. 2

EP 4 123 656 A1

**Description**

FIELD OF THE INVENTION

The present invention relates to the field of medical records, and in particular to the identification of relevant sections of medical records.

BACKGROUND OF THE INVENTION

There is an increasing use and reliance upon medical records of a subject during the course of clinical assessment and analysis of the subject. Moreover, there is an increasing amount of unstructured textual data being stored in medical records, as it is becoming more common for written reports of medical treatments or analysis to be recorded to minimize the chances of pre-existing medical treatments or analysis being missed (e.g. and unnecessarily repeated or otherwise not taken into account, e.g. for adjusting or personalization therapy or treatment).

The significant increase in the amount of unstructured text within medical records has created a significant burden for any clinician taking over responsibility for a subject. At present, such a clinician would need to review the subject's entire medical history to make sure that they identify all relevant information, i.e. to not miss anything of medical importance. As a result, there can be a significant delay between either a clinician reacquainting themselves or a (new) clinician taking over responsibility of a subject and any subsequent treatment or analysis of the subject taking place.

It would be desirable to reduce the amount of information that a clinician needs to read or take account of, in order to speed up time before treatment or further assessment of the subject and prevent clinicians from being (partially) uninformed on a patient's medical status and history as due to the large amount of data available on patients in hospital information system clinicians do not have, or are not able to take the time to get fully acquainted with a patient's medical status and history.

One approach could be to use an automatic highlighting mechanism, which processes the textual data using a machine-learning algorithm (or the like) to identify key portions of the textual data. These key portions may then form a summary of the textual data.

Another approach could be a fully manually summarization approach, where a user or clinician reads through the textual data and manually extracts medically relevant parts to form a summary of the textual data.

There is therefore an ongoing desire to improve mechanisms for identifying (medically) relevant parts of unstructured textual data of a medical record.

SUMMARY OF THE INVENTION

The invention is defined by the claims.

According to examples in accordance with an aspect of the invention, there is provided a computer-implemented method of estimating the relevance of different sections of a medical record of a subject. The computer-implemented method comprises: obtaining the medical record of the subject containing unstructured textual data; dividing the unstructured textual data into a plurality of sections, each section containing a different part of the unstructured textual data; providing a visual representation of the unstructured textual data at a first user interface; visually emphasizing different sections of the unstructured textual data, at the first user interface, responsive to a user input; monitoring, for each section of the unstructured textual data, at least one characteristic of the section of the unstructured textual data, wherein the at least one characteristic comprises a characteristic dependent upon at least one visual emphasization of the section; and for each section of the unstructured textual data, processing the monitored characteristics to estimate a relevance of the section of the unstructured textual data.

The present invention proposes a new approach for identifying a relevance of different parts of unstructured textual data of a medical record. The textual data is (sub)divided into a plurality of sections. Different parts of the textual data are visually emphasized at a user interface responsive to a user input. A relevance of each section is determined based on one or more characteristics of the said section. The one or more characteristics includes at least one characteristic that is dependent upon a visual emphasization of the said section (resulting from the user input). Thus, the user input defines which section(s) is visually emphasized, which defines characteristics of the textual data with respect to the user input.

The proposed approach facilitates identification of a relevance of different sections of unstructured textual data that relies upon analysis of user interactions with the sections, without the need for the user to explicit flag or identify that a section is relevant. The invention relies upon an assumption that a visually emphasized section is the section most likely to be the section that holds the user's attention. The more times or the longer that a section holds the user's attention, the more likely that said section is to be (medically) relevant. As another example, if a user repeatedly returns to a particular section, then it is more likely that said section is of medical relevance or importance.

The present invention recognizes that characteristics responsive to a visual emphasisation of a section can therefore reflect a perceived relevance or importance of that section. In this way, a relevance of a section can be estimated, e.g. for use in later constructing a summary containing relevant sections (e.g. a subset of the most relevant sections).

Thus, the present invention is able to identify a relevance of different sections by assessing the interaction between a user of the textual data and the sections of the textual data, and in particular, characteristics that result from how each section is visually emphasized. The (semi-automatic) identification of relevant text in medical records facilitates improved speed, ease and accuracy of identifying medically relevant text (compared to existing approaches). This directly leads to improved patient outcome, by reducing an amount of time that a clinician needs to identify relevant sections and reducing a likelihood that medically relevant information will be missed. In particular, summaries of unstructured textual data can be generated that contain information that might have been missed by fully automated protocols.

The relevance of a section may be represented by a numeric, binary or categorical indicator. A numeric indicator is a measure of relevance of the section, e.g. on a scale of 0 to 1, 0 to 10, 0 to 100, 1 to 10 or 1 to 100. Other scales will be apparent to the skilled person. A binary indicator may indicate whether or not the section is relevant. A categorical indicator may indicate a category of relevance, e.g. "Highly Relevant", "Relevant", "Neither Relevant or Irrelevant", "Irrelevant", "Highly Irrelevant". Other suitable categories or labels will be apparent to the skilled person.

Examples of user input for causing the visual emphasizations of different sections will be apparent to the skilled person. For instance, a user may scroll through the different identified sections to visually emphasize the different sections in turn. As another example, the user may select (e.g. click on) different sections to result in their visual emphasisation. As yet another example, a user may search (using a search tool) for a particular keyword, and any section including the keyword may be visually emphasized.

In some embodiments, the plurality of sections of the unstructured textual data forms a sequence of sections; and the step of visually emphasizing different sections of the unstructured textual data comprises visually emphasizing different sections in sequential order, wherein the movement through the sequence is responsive to the user input.

Thus, the visual emphasisation may effectively scroll through the different sections. This approach mimics a reading style of a user, and therefore more accurately represents how a user reads the report.

In some examples, the at least one characteristic further comprises a user identification of relevance of the section.

The at least one characteristic may comprises a characteristic responsive to a length of time that the section of unstructured textual data is visually emphasized. The longer that a user reads a section, the more relevant said section is likely to be for a summary of the medical record. This effectively allows the system to estimate whether a user actually reads or merely skims the text.

The at least one characteristic may comprise a characteristic responsive to a length of time that the section of unstructured textual data is visually emphasized and a measure of size of the section of unstructured textual data. The measure of size of the section may be a number of words, a number of letters, a number of syllables, a number of predetermined phrases and so on.

In some examples, the at least one characteristic comprises a characteristic responsive to a measure of size of the section of unstructured textual data divided by the length of time that the section of unstructured textual data is visually emphasized. This embodiment recognizes that a reading time will naturally vary depending upon the length of the sections (in addition to its relevance and/or importance). By taking the length of the section into account, more useful data for assessing the relevance of the section will be generated.

Optionally, the at least one characteristic comprises a characteristic responsive to a number of times that the user(s) causes the section of unstructured textual data is visually emphasized.

In at least one embodiment, the steps of providing a visual representation of the unstructured textual data and visually emphasizing different sections of the unstructured textual data are repeated for each of a plurality of different users; and the at least one characteristic comprises a number of different users that cause the section to be visually emphasized.

Some embodiments are adapted wherein the step of dividing the unstructured textual data comprises dividing unstructured textual data into a plurality of super-sections, each super-section containing a plurality of sections of unstructured data; and wherein the at least one characteristic of the section of the unstructured textual data comprises: a number of times that the super-section in which the section is contained is viewed by the user; and/or an identification of whether or not the section is contained in the last super-section viewed by the user before the user ends their interaction with the unstructured medical data.

Different super-sections may effectively represents different pages, paragraphs or chapters of the unstructured textual data. Approaches for identifying that a super-section is viewed (at any given moment in time) will be readily apparent to the skilled person, e.g. by tracking which super-section is displayed at an uppermost part of the area of the user interface providing the visual representation of the textual data or by tracking which super-section occupies the largest part of the area of the user interface providing the visual representation of the textual data. Other approaches will be apparent to the skilled person.

In at least one example, the at least one characteristic of the section of the unstructured textual data comprises a proportion of time that the section is visually emphasized with respect to the total time that the user interacts with the medical record or the unstructured medical data.

The step of visually emphasizing different sections of the unstructured textual data may comprise visually emphasizing a section by performing one or more of the following: highlighting the section of unstructured textual data; changing a font size of the section of unstructured textual data; changing a kerning and/or letter-spacing of the section of unstructured textual data; changing a color of the section of unstructured textual data; changing a typographic emphasis of the section of unstructured textual data; moving the section of unstructured textual data according to a movement pattern; and/or inserting additional space at one or both ends of the section of unstructured textual data.

Some embodiments further comprise selecting a subset of the sections of the unstructured textual data responsive to estimated relevance of each section, wherein the subset does not include all of the sections of the unstructured textual data; and generating a summarized medical record containing the selected subset of the sections of the unstructured textual data, wherein the summarized medical record does not include all of the sections of the unstructured textual data.

The computer-implemented method may further comprise a step of displaying the summarized medical record at a second user interface.

There is also proposed a computer program product comprising computer program code means which, when executed on a computing device having a processing system, cause the processing system to perform all of the steps of any herein described method.

There is also proposed a processing system configured to estimate the relevance of different sections of a medical record. The processing system is configured to: obtain the medical record of the subject containing unstructured textual data; divide the unstructured textual data into a plurality of sections, each section containing a different part of the unstructured textual data; provide a visual representation of the unstructured textual data at a first user interface; visually emphasize different sections of the unstructured textual data, at the first user interface, responsive to a user input; monitor, for each section of the unstructured textual data, at least one characteristic of the section of the unstructured textual data, wherein the at least one characteristic comprises a characteristic dependent upon at least one visual emphasization of the section; and for each section of the unstructured textual data, process the monitored characteristics to estimate a relevance of the section of the unstructured textual data.

The processing system may be configured to perform any herein described method, and vice versa.

There is also proposes a system comprising the processing system and the first user interface. The system may further comprise a memory from which the processing system obtains the medical record of the subject.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 illustrates an overview of an approach;
Figure 2 is a flowchart illustrating a method;
Figure 3 is a flowchart illustrating another method;
Figure 4 is a flowchart illustrating a further method;
Figure 5 illustrates a processing system; and
Figure 6 illustrates a system comprises a processing system.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for estimating the relevance or importance of sections of unstructured (medical) textual data. Different sections are visually emphasized responsive to a user input, and one or more characteristics of when the sections are visually emphasized are monitored. Other characteristics of the sections may also be monitored or determined. These characteristics are then processed to predict or determine a relevance of each section.

One underlying concept is based on the realization that a reader of textual data will pay attention to a visually emphasized section. Thus, if a user is able to change which sections are visually emphasized, a user's attention to any given section can be tracked by tracking which sections are visually emphasized. It is herein recognized that this information can be used to (semi-)automatically determine which sections are of relevance, as these will be the sections to which a user pays most attention (which will be indicated in the monitored characteristics).

Embodiments may be employed in any clinical environment in which medical records contain substantial amounts of unstructured textual data, in order to facilitate the generation of a summarized or reduced version of the unstructured textual data or to highlight key or important sections of the unstructured textual data.

In the context of the present invention, a medical record is any (stored) data that contains information on a patient that may affect a health and/or diagnosis of the patient. For instance, a medical record may contain clinical notes, laboratory test and analysis data, clinical observations, past medical history, family history, lifestyle information, health questionnaire information and so on.

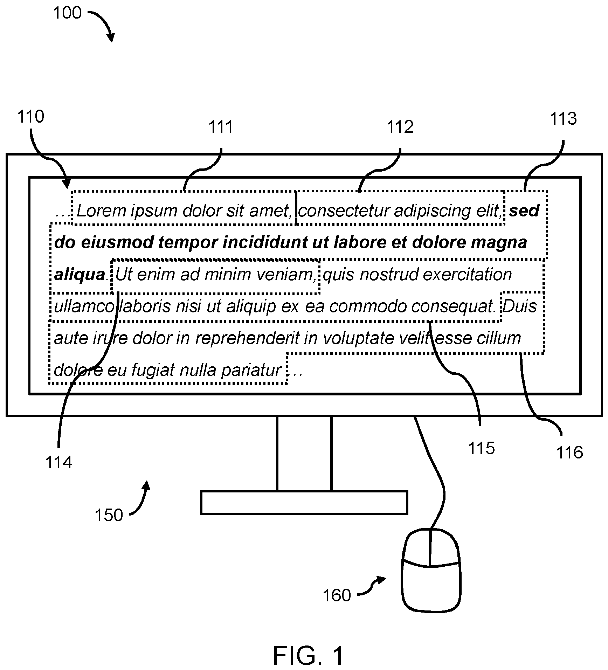

Figure 1 illustrates a conceptual overview of an approach 100 proposed in the present disclosure.

Unstructured textual data 110 of a medical record is subdivided into a plurality of parts, segments or sections 111-116. Each section may represent a paragraph or sub-paragraph of text of the unstructured textual data. For instance, each section may represent a different sentence, clause or phrase of the unstructured textual data. In the illustrated example, each section here represents a phrase of a sentence, where each phrase comprises any text spanning between two punctuation marks.

A portion of the unstructured textual data is presented to a user via a display 150. One section 111-116 of the unstructured medical text at a time is visually emphasized to the user, e.g. through the use of highlighting, color, transparency/opacity, size and so on. In the illustrated example, the currently visually emphasized section is section 113. A user input, e.g. at an input interface 160, defines which section is visually emphasized. This can be performed, for instance, by the user clicking or selecting a particular section or by the user sequentially scrolling through the different sections.

At least one characteristic of each section is monitored, where each characteristic is dependent upon a visual emphasization of the section. In particular, each characteristic may be a characteristic that changes responsive to that section becoming visually emphasized or stopping being visually emphasized, i.e. a characteristic that changes responsive to when (i.e. the time(s) at which) the section is visually emphasized or stops being visually emphasized. Example characteristics include a number of times the section is visually emphasized, a length of time for which a section is visually emphasized, a frequency at which the section is visually emphasized and so on. Further suitable examples will be provided later in this disclosure.

The monitored characteristics can then be processed to determine a relevance of each section, e.g. an importance of the section in representing the overall medical report or unstructured textual data.

The present disclosure is based upon the realization that information responsive to when a section is visually emphasized represents a user's attention to that section. Thus, tracking of a user's attention to a particular section (and therefore a relative importance of that section) can be performed by monitoring when the section is visually emphasized. This is because it can be assumed that a currently visually emphasized section is the section to which a user pays attention.

In the context of the present disclosure, a relevance is a relative perceived importance of information contained in a section to a clinician's understanding of the patient's condition and/or medical history. In particular, a relevance of a section is greater if a user is more likely to take the information contained in the section into account when determining how to treat, assess or diagnose the subject.

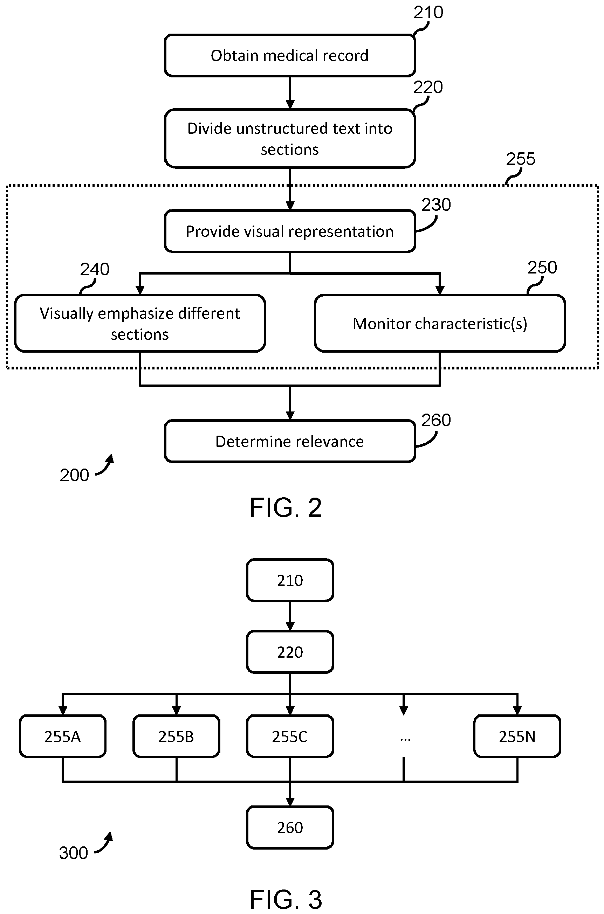

Figure 2 illustrates a method 200 according to an embodiment of the invention. The method 200 provides an approach for estimating the relevance of different sections of a medical record of a subject. The method may be performed by a processing system (not shown).

The method 200 comprises a step 210 of obtaining the medical record of the subject containing unstructured textual data. The medical record may be obtained from a storage unit, e.g. a memory storage. Step 210 may be performed by an input interface of a processing system carrying out method 200.

The unstructured textual data comprises (e.g. descriptive) text that is not (formally) arranged or organized in a pre-defined configuration. Suitable content for unstructured textual data may include medical reports, medical notes, questionnaire responses, and/or textual descriptions of the subject and/or their condition.

The method then perform a step 220 of dividing the unstructured textual data into a plurality of sections, each section containing a different part of the unstructured textual data. The division of the unstructured textual data into a plurality of sections may comprise splitting the unstructured textual data by paragraph, sentence, phrase, clause and/or (display) line. In other examples, the unstructured textual data may be divided into sections having a fixed or predetermined length (e.g. predetermined number of words, letters or sentences). Other suitable divisions for unstructured textual data will be apparent to the skilled person.

In some examples, the length of each section may be no less than 20 words. Preferably, the length of each section is no greater than a maximum sentence length of the unstructured textual data and/or no greater than 50 words. This approach ensures that more specific identification of relevant text can be performed.

It will be apparent that the plurality of sections forms a sequence of sections, as the sections are ordered in accordance to their position in the original (undivided) unstructured textual data.

The method then performs a step 230 of providing a visual representation of the unstructured textual data at a first user interface. The first user interface may, for instance, be a display screen or other visual output device. Step 230 may be performed in response to a user input selecting or opening the unstructured textual data.

In this way, the unstructured textual data is presented to a user to facilitate interaction between the unstructured textual data and the user. The user may be able to end their interaction with the unstructured textual data (e.g. by closing the visual representation of the unstructured textual data).

The method then performs a process 240 of visually emphasizing different sections of the unstructured textual data, at the first user interface, responsive to a user input. In particular, process 240 may comprise receiving a user input indicating a desired change to which section is visually emphasized, and controlling the visual representation of the unstructured textual data at the first user interface to visually emphasize a different section.

The visually emphasized section may be surrounded by other (non-visually emphasized) sections before and/or after the visually emphasized section in the sequence of sections, which can help provide contextual understanding for the visually emphasized section. Not all sections of the unstructured textual data need to be displayed at once, rather only the visually emphasized section should be displayed (preferably, alongside sections proximate to the visually emphasized section in the sequence).

The user input may be provided by a user input interface that generates electrical signals responsive to actions of the user. Examples of these are well known to the skilled person, and include computer mice, keyboards, touch-screen interfaces and so on.

The user input may, for instance, be an indication to move to the next section in the plurality of sections. Thus, as the plurality of sections forms a sequence of sections, the user input may be an indication to move to the next section in the sequence of sections. This user input may, for instance, be in the form of a scroll input received from an input interface.

Thus, the plurality of sections of the unstructured textual data may form a sequence of sections; and the step of visually emphasizing different sections of the unstructured textual data may comprise visually emphasizing different sections in sequential order, wherein the movement through the sequence is responsive to the user input. In this instance, the user input may be a scroll indication (e.g. provided by a movement of a scroll wheel of a mouse or pressing of an upward or downward arrow on a keyboard).

As another example, the user input may be an indication of a desired section to be visually emphasized. For instance, the user (clinician) may select or click on a non-visually emphasized section to indicate a desire to visually emphasize the section.

As yet another example, the user input may be an indication an identity of the desired section. For instance, each section may be associated with a different number, and a user may input a number to jump to a particular section.

Visually emphasizing of a section may comprise highlighting the section (e.g. changing a background color of a section), adjusting a color of a section, inducing a (slight) movement of the section, changing a font style or size of the section (e.g. using non-rounded characters for only the visually emphasized section), extending the whitespace around the visually emphasized section and so on.

Other approaches for visually emphasizing a section may comprise modifying an appearance of other sections adjacent to or surrounding the visually emphasized section, e.g. adjusting a color of one or more surrounding sections, inducing a (slight) movement of the surrounding section(s), changing a font style or size of the surrounding section(s) (e.g. using rounded characters for only the non-visually emphasized section), and so on.

Other approaches for visually emphasizing a section include: changing a kerning and/or letter-spacing of the section of unstructured textual data; changing a typographic emphasis of the section of unstructured textual data; moving the section of unstructured textual data and/or text surrounding the section according to a movement pattern; and/or inserting additional space at one or both ends of the section of unstructured textual data.

A combination of one or more of any herein described approaches may be used to visually emphasize a section.

Generally, visually emphasizing a section means changing one or more characteristics of the displayed section (and/or surrounding sections or text) to visually distinguish the visually emphasized section from other sections. The precise mechanism by which a section is visually emphasized (or not visually emphasized) is immaterial.

The method 200 also performs a process 250 of monitoring, for each section of the unstructured textual data, (e.g. values of) at least one characteristic of the section of the unstructured textual data. The at least one characteristic comprises one or more characteristics dependent upon at least one visual emphasization of the section, i.e. comprises one or more emphasization-dependent characteristics. Process 250 is performed alongside process 240, e.g. whilst process 240 is being performed.

As previously explained, the emphasization-dependent characteristic (s) may be a characteristic that changes responsive to that section becoming visually emphasized or stopping being visually emphasized, i.e. a characteristic that changes responsive to when (i.e. the time(s) at which) the section is visually emphasized or stops being visually emphasized.

This may include, for instance, a number of times that the section is visually emphasized, a number of times that the section is visually emphasized for more than predetermined time period, a total amount of time that the section is visually emphasized, a number of times that the section is revisited by the user and so on.

Another example of an emphasization-dependent characteristic is an identification of whether or not the section is the last section that is visually emphasized before the user ends their interaction with the unstructured textual data. The last visually emphasized section may represent a section of relevance (e.g. as it may contain information that answers a user's enquiry or reason for investigating the unstructured textual data, leading them to stop interacting with the unstructured textual data).

Step 230 and processes 240, 250 form a combined process 255. The combined process represents a process for obtaining one or more characteristics of each section of unstructured textual data, including at least one emphasization-dependent characteristic, for one instance of a visual representation of the unstructured textual data provided to a single user.

The method 200 then performs a step 260 of, for each section of the unstructured textual data, processing (at least) the monitored characteristic(s) to estimate a relevance of the section of the unstructured textual data.

Thus, step 260 comprises processing at least information about how a section is visually emphasized in order to estimate a relevance of that section. In other words, step 260 produces, for each section, an estimated relevance by processing at least one emphasization-dependent characteristic (and optionally one or more other characteristics) of the section.

Purely by way of example, the more times that a section is visually emphasized, the greater the relevance of that section (e.g. as it can be assumed that repeated reading of this section will take place, and therefore is of more relevance). As another example, the greater the amount of time that a section is visually emphasized (e.g. relative to the length of the section) may indicate that the section is of greater relevance.

The estimated relevance may be in the form of an indicator, which may be binary, numeric or categorical. By way of example, the estimated relevance may take the form of a numeric indicator or measure, e.g. on a predetermined scale. The numeric indicator may represent an estimated relevance of the section.

One or more formulae or rules may be used to process the monitored characteristics in order to produce the numeric indicator.

By way of simple example, if the monitored characteristic includes (for each section) a measure $T_s$ of how many times that the segment is visually emphasized for more than a predetermined time period, then a relevance $Re(s_n)$ of the section may be defined by:

$$Re(s_n) = \frac{T_s}{TT_s} \qquad (1)$$

where $TT_s$ represents a total number of times that any segment of the unstructured text is visually emphasized for more than the predetermined time period. Thus, in this embodiment, the more times that a segment is emphasized (for a predetermined time period), then the greater the perceived relevance of the segment.

The value of $TT_e$ provides a mechanism for normalizing the value $T_e$. In some examples, the value $T_e$ may itself act as an indicator of estimated relevance.

As another example, if the monitored characteristic(s) includes (for each section) a measure $t_s$ of a length of time for which that section is visually emphasized, then a relevance $Re(s_n)$ of that section may be defined by:

$$Re(s_n) = \frac{t_s}{L_s . Tt_S} \qquad (2)$$

where $L_s$ is a length of that section (e.g. in words, letters or syllables) and $Tt_s$ is a total amount of time that any section is visually emphasized (e.g. a total length of time that a visual representation of the unstructured textual data is provided). In this example, the greater the length of time that a section is visually emphasized, then the greater

In some examples, the one or more formulae comprise at least one weighted formulae.

For instance, if the monitored characteristics include $T_s$ and $t_s$, then a relevance $Re(s_n)$ can be estimated as:

$$Re(s_n) = w_1 \frac{T_s}{TT_s} + w_2 \cdot \frac{t_s}{L_s . Tt_S} \tag{3}$$

where $w_1$ and $w_2$ are (predetermined) weights.

The above embodiments are only examples of how a measure of relevance can be determined from characteristics of a section, where the characteristics include at least one emphasization-dependent characteristic. Any combination of different characteristics (such as those herein described) may be used to determine a relevance of a section.

Other forms of indicator for the estimated relevance are also contemplated, e.g. a binary indicator (e.g. a "1" or "0" representing relevance or irrelevance) or a categorical indicator (e.g. where different categories represent different categories of relevance).

In some examples, a machine-learning method is used to process the monitored characteristics (and optionally, additional information) in order to produce the measure of relevance. Thus, the machine-learning method may receive, as input, one or more (values of) characteristics of a section and provide, as output, a predicted relevance of the section.

In the previously described embodiment, a relevance of each section is determined based upon the actions of a single user (i.e. how a section is visually emphasized responsive to the user input). In other words, combined process 255 is performed for only a single user.

However, step 260 can be modified to use monitored characteristics of the visual representation to a plurality of different users (e.g. at a same or different user interface). In other words, combined process 255 may be repeated for a plurality of users, and the monitored characteristics from all of the users may be processed in step 260.

This embodiment is illustrated in Figure 3, which illustrates a method 300 in which a plurality of combined processes 255A, 255B, 255C ... 255N are performed, each being a different instance of process 255 described in Figure 2. Each process provides a visual representation of the unstructured text to a different user (e.g. at different user interfaces and/or at a same user interface at different points in time). Each instance of combined process 255 provides monitored characteristics of each section for different users.

This embodiment provides additional information that can be used to assess the relevance of each section. For instance, a (total) number of users that visually emphasize a particular section can be used in the assessment of the relevance of that section, where a greater number of users that visually emphasize a section indicate an increased perceived relevance of that section. As another example, an average time that a section is visually highlighted by the users can represent another characteristic. Equations (1) to (3) may be modified accordingly.

Thus, the at least one characteristic of a section processed in step 260 may comprise a number of users that cause the section to be visually emphasized.

The method 300 facilitates a plurality of users (e.g. care providers) involved in care for a particular subject to contribute to the identification of relevant sections of the unstructured textual data This enables a more collaborative approach and improves alignment amongst care providers involved. This embodiment also reduces the likelihood that important information will be considered irrelevant, as a wider range of users (e.g. having different experience levels, specialties, training and so on) are able to effectively contribute to the assessment of relevance.

With reference to both Figures 2 and 3, and as previously explained step 260 is not limited to using just the emphasization-dependent characteristic(s) of the visual emphasisation to assess an importance. Rather, other characteristics of a section may be processed in the estimation of a relevance of a section.

For instance, the at least one characteristic further comprises a user identification of relevance of the section. The user may be able to provide an indication of relevance of a section (e.g. provide a manual marker of relevance). In some examples, this can be achieved by providing (alongside a visual representation of any given section) a checkbox with which the user can interact. Interaction of the user with the checkbox may indicate that the associated section is relevant. Other forms of user input for marking sections as relevant will be apparent to the skilled person (e.g. if a user highlights a particular section or copies a particular section).

In some examples, if a user has identified that a section is relevant (e.g. via an appropriate user input, step 260 may estimate the relevance of the section to be a maximum possible relevance. For instance, if a relevance is a numeric measure generated on a scale of 0 to 1, then the relevance may be set to 1 if the user has identified the section as relevant.

In other words, a direct user indication that a section is relevant may override any other determination of the relevance of a section. If no direct user indication is received, then another approach to estimating the relevance of a section may be performed. The direct user indication could be used as a labelled input to a method or funtion to calibrate a function or model (as described earlier could be used to calibrate weights and/or thresholds).

In some examples, each section of the text belongs to one or more super-sections. A super-section represents a larger body of text than contained in a single section, and may comprise (for instance) a paragraph and/or page of the text.

Unstructured textual data can be pre-divided into a plurality of super-sections, e.g. pages or paragraphs. Information responsive to a user interaction with the super-section to which a section belongs provides additional information that could indicate a potential relevance of the section. In particular, the more that a user interacts with a particular super-section (e.g. views a page), the more likely that a section in that super-section is to be of relevance.

The one or more characteristics of a section (processed in step 260) may comprise a number of times that the super-section in which the section is contained is viewed by the user; and/or an identification of whether or not the section is contained in the last super-section viewed by the user before the user ends their interaction with the unstructured medical data.

Different super-sections may therefore effectively represents different pages, paragraphs or chapters of the unstructured textual data. Approaches for identifying that a super-section is viewed (at any given moment in time) will be readily apparent to the skilled person, e.g. by tracking which super-section is displayed at an uppermost part of the area of the user interface providing the visual representation of the textual data or by tracking which super-section occupies the largest part of the area of the user interface providing the visual representation of the textual data. Other approaches will be apparent to the skilled person.

These characteristics provide information that demonstrate a contextual relevance of the section of text.

The skilled person would be capable of developing a method for using any combination of one or more characteristics previously described, including at least one emphasization-dependent characteristic, to estimate the relevance of a section. Thus, step 260 can be performed using any number of plausible approaches.

In one working example, step 260 comprises estimating a relevance Re(.) of a section $s_n$ using the following functions:

$$\text{Re}(s_n) = \begin{cases} 1 & \text{if } M(s_n) = 1 \\ W_{\text{users}} \cdot N_{\text{users}} \cdot \left(\dfrac{W_T \cdot T}{L}\right) + W_{rv} \cdot N_{rv} + W_{pv} \cdot N_{pv} & \text{if } M(s_n) = 0 \end{cases} \tag{4}$$

in which:

$M(s_n)$ is an indication of whether ($M(s_n)$ = "1") or not ($M(s_n)$ = "0") a user has (manually) indicated the section's relevance via a user input;

$N_{\text{users}}$ is a ratio or proportion of users that cause section $s_n$ to become visually emphasized or the ratio or proportion of users that cause section $s_n$ to become visually emphasized for more than a predetermined period of time;

T is the length of time for which the section is visually emphasized, this may be the total amount of time across all users for which data is collected or the average amount of time across the users;

L is the number of words (in the section) divided by average word length (of words in the section, in the super-section, in the unstructured textual data or globally (e.g. for the language used in the unstructured textual data);

$N_{rv}$ is the number of times the section is revisited, i.e. a number of times that any user causes the section to become visually emphasized (e.g. for more than a predetermined period of time) at least twice during a reading of the unstructured text; and

$N_{pv}$ represents a number of page views, i.e. a number of times the super-section containing the section is viewed.

The values $W_x$ (where x can change) are the weights related to the various determinant. The values of the weights may be automatically selected and/or adjusted so that sections that are marked (by the user) as being relevant would have (if processed using the function with the weights), e.g. on average, a higher relevance value than other sections, preferably wherein a maximum relevance is equal to 1. This facilitates automated identification of potentially relevant sections.

Another example for performing step 260 processes or combines time spent on the section and the user(s) exit points, e.g. which identify the section that the user(s) ends their interaction with the visual representation of the unstructured text (e.g. closes the display of unstructured text). This embodiment relies upon a recognition that a user would end their interaction (immediately) after reading a section with relevant information. In this way, relevance of a section is based on time spend on the section and whether or not users exited after this section, e.g. following the following equation:

$$\text{Re}(s_n) = \begin{cases} 1 & \text{if } M(s_n) = 1 \\ W_{\text{users}} \cdot N_{\text{users}} \cdot \left(\dfrac{W_T \cdot T}{L}\right) + W_{\text{exit}} \cdot N_{\text{exit}} & \text{if } M(s_n) = 0 \end{cases} \tag{5}$$

in which $N_{exit}$ represents the number of times (across all users) that a user ends their interaction with the unstructured text whilst the section is visually emphasized and/or within a predetermined period of time since the section was last visually emphasized.

Optionally, the formula used when $M(s_n)$ is 0 (for either equation (4) or (5) or other similar equations) further comprises a normalization process, so that the maximum value output by the normalized formula is equal to 1. Equations (4) and (5) may be modified so that the equation used when $M(s_n)$ is 0 is instead performed for all sections (i.e. manual indications are ignored).

Another approach for performing step 260 is to obtain patterns of visual emphasisation (i.e. a history of how sections are visually emphasized for each user) and looking for patterns or overlaps between various users as a measure of section relevance. Thus, a relevance of sections could be based on how users navigate the unstructured text as a whole (as indicated by the sequence in which sections are visually emphasized). Monitoring a movement from one section to the next allows the journey that a user takes through the text to be recorded and analyzed. Comparing different user's journeys enables identification of overlapping journey segments, which can be inferred as containing sections of relevance - as they indicate parts of the text that have been re-read. Recurrence of journey patterns in various users can thereby be used as a measure of relevance. For instance, the number of times a same journey segment occurs for all users is a measure for relevance (of sections included in the journey segment). This approach also facilitates identification of links or connections between different sections. If many users move from a first section to a second section, it can be inferred that there is a relevant link between these two sections. This information can be used to share or influence the relevance of each section and may, as later described be used to place these sections together in a summary text for improved clarity.

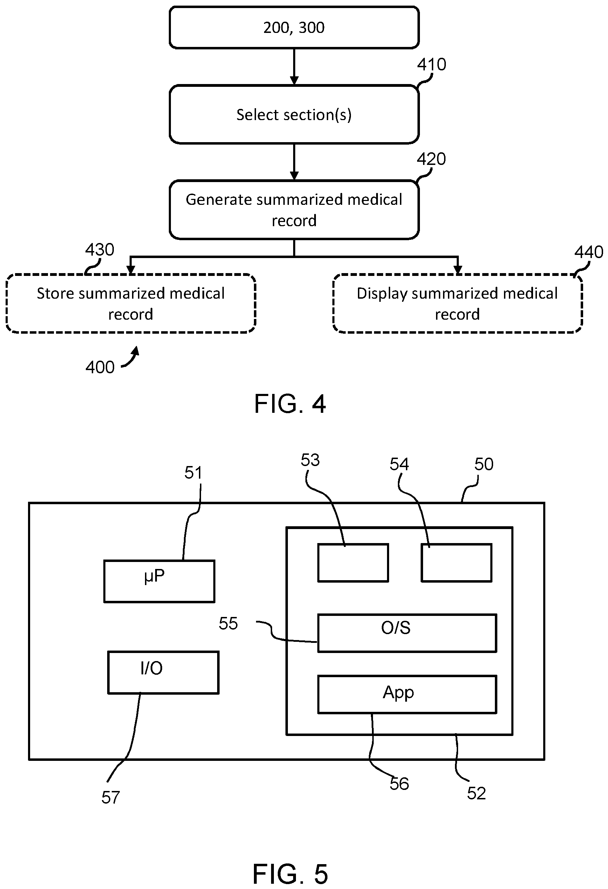

Figure 4 illustrates a method 400 according to a further embodiment of the invention. The method 400 provides an approach for generating a summarized medical record.

The method 400 comprises performing the process 200, 300 previously described, in order to determine a relevance of each section of the unstructured textual data.

The method 400 then moves to a step 410 of selecting a subset (i.e. not all) of the section of the unstructured textual data to include in the summarized medical report. The subset is responsive to the estimated relevance of each section.

By way of example, where the estimated relevance of each section is a numeric indicator on a predetermined scale, step 410 may comprise selecting each section having a numeric indicator that breaches (e.g. exceeds or meets) some predetermined threshold.

The predetermined threshold may, for example, be defined so that sections marked (by the user) as relevant are selected. Where the sections that are not marked by the user are processed according to some predetermined function to generate a relevance value, the predetermined threshold may be selected so that sections marked by the user as relevant would (if processed according to the predetermined function) be selected, e.g. the predetermined threshold may be selected to be the lowest value amongst values of sections (marked as relevant) that are processed according to the predetermined function.

As another example, where the estimated relevance of each section is a binary indicator, step 410 may comprise selecting all sections for which the binary indicator indicates that the section is predicted to be relevant.

The method 400 further comprises a step 420 of generating a summarized medical record containing the selected subset of the sections of the unstructured textual data, wherein the summarized medical record does not include all of the sections of the unstructured textual data.

Thus, only some of the unstructured textual data is included in the summarized medical record. The included sections may thereby effectively represent an abstract of the most relevant parts of the unstructured textual data of the medical record.

The summarized medical record comprise additional information, e.g. acquired from structured data or medical information. By way of example, the summarized medical record may comprise dated information extracted from the medical history on medication, treatments, laboratory results and so on.

In some examples, if links between different sections of the unstructured textual data have been identified, step 420 may comprise controlling the summarized medical record so that linked sections of the unstructured textual data (including in the summarized medical report) are placed adjacent to one another.

The summarized medical record may be stored, e.g. for later access by a clinician or user. Thus, the method may comprise a step 430 of storing the summarized medical record, e.g. in a memory or database. The memory or database may be the same memory or database from which the medical record was originally obtained, or a memory (e.g. dedicated to summarized medical records).

In some examples, the method 400 further comprises a step 440 of displaying the summarized medical record at a second user interface. The displayed medical record thereby provides a clinician (interacting with the second user interface) with information required to assess the condition of the subject, and is smaller in size than the unstructured textual data.

In this way, the clinician is provided with a smaller amount of data to process and analyze, with a low likelihood that important data will be missed (as the selected data is dependent upon previous clinicians or users reading of the unstructured textual data).

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

By way of further example, Figure 5 illustrates an example of a computer 50 within which one or more parts of an embodiment may be employed. Various operations discussed above may utilize the capabilities of the computer 50. For example, one or more parts of a system estimating a relevance of sections of unstructured textual data of a medical record may be incorporated in any element, module, application, and/or component discussed herein. In this regard, it is to be understood that system functional blocks can run on a single computer or may be distributed over several computers and locations (e.g. connected via internet).

The computer 50 includes, but is not limited to, PCs, workstations, laptops, PDAs, palm devices, servers, storages, and the like. Generally, in terms of hardware architecture, the computer 50 may include one or more processors 51, memory 52, and one or more I/O devices 57 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 51 is a hardware device for executing software that can be stored in the memory 52. The processor 51 can be virtually any custom made or commercially available processor, a central processing unit (CPU), a digital signal processor (DSP), or an auxiliary processor among several processors associated with the computer 50, and the processor 51 may be a semiconductor based microprocessor (in the form of a microchip) or a microprocessor.

The memory 52 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 52 may incorporate electronic, magnetic, optical, and/or other types of storage media. Note that the memory 52 can have a distributed architecture, where various components are situated remote from one another, but can be accessed by the processor 51.

The software in the memory 52 may include one or more separate programs, each of which comprises an ordered listing of executable instructions for implementing logical functions. The software in the memory 52 includes a suitable operating system (O/S) 55, compiler 54, source code 53, and one or more applications 56 in accordance with exemplary embodiments. As illustrated, the application 56 comprises numerous functional components for implementing the features and operations of the exemplary embodiments. The application 56 of the computer 50 may represent various applications, computational units, logic, functional units, processes, operations, virtual entities, and/or modules in accordance with exemplary embodiments, but the application 56 is not meant to be a limitation.

The operating system 55 controls the execution of other computer programs, and provides scheduling, input-output control, file and data management, memory management, and communication control and related services. It is contemplated by the inventors that the application 56 for implementing exemplary embodiments may be applicable on all commercially available operating systems.

Application 56 may be a source program, executable program (object code), script, or any other entity comprising a set of instructions to be performed. When a source program, then the program is usually translated via a compiler

---

(such as the compiler 54), assembler, interpreter, or the like, which may or may not be included within the memory 52, so as to operate properly in connection with the O/S 55. Furthermore, the application 56 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 57 may comprise at least an input interface. The input interface is configured to obtain or receive data to the processing system and may, for instance, be configured to obtain the unstructured textual data (e.g. from an external device, such as a medical record system).

The I/O devices 57 may also comprise an output interface, which is configured to provide data for controlling an external device or passing to an external device. By way of example, the output interface may provide estimated relevances and/or summarized medical records to an external device (e.g. for further processing) and/or provide display data for controlling a display to provide a visual representation of the summarized medical records and/or estimated relevances.

If the computer 50 is a PC, workstation, intelligent device or the like, the software in the memory 52 may further include a basic input output system (BIOS) (omitted for simplicity). The BIOS is a set of essential software routines that initialize and test hardware at startup, start the O/S 55, and support the transfer of data among the hardware devices. The BIOS is stored in some type of read-only-memory, such as ROM, PROM, EPROM, EEPROM or the like, so that the BIOS can be executed when the computer 50 is activated.

When the computer 50 is in operation, the processor 51 is configured to execute software stored within the memory 52, to communicate data to and from the memory 52, and to generally control operations of the computer 50 pursuant to the software. The application 56 and the O/S 55 are read, in whole or in part, by the processor 51, perhaps buffered within the processor 51, and then executed.

When the application 56 is implemented in software it should be noted that the application 56 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 56 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

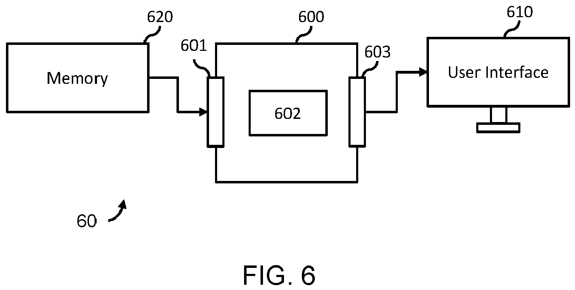

Figure 6 illustrates a system 60 according to an embodiment.

The system 60 comprises a processing system 600, which may be embodied as the processing system 50 described with reference to Figure 5. Other suitable examples of a processing system will be apparent to the skilled person.

The system 60 may also comprise a (first) user interface 610.

The processing system 600 may comprise an input interface 601, configured to obtain the medical record of the subject containing unstructured textual data.

The processing system 600 may obtain the medical record of the subject from a memory or database 620, which forms an optional part of the system 60.

The processing system 600 may comprise a processor 602 configured to divide the unstructured textual data into a plurality of sections, each section containing a different part of the unstructured textual data; provide a visual representation of the unstructured textual data at a first user interface 620; visually emphasize different sections of the unstructured textual data, at the first user interface, responsive to a user input; monitor, for each section of the unstructured textual data, at least one characteristic of the section of the unstructured textual data, wherein the at least one characteristic comprises a characteristic dependent upon at least one visual emphasization of the section; and for each section of the unstructured textual data, process the monitored characteristics to estimate a relevance of the section of the unstructured textual data.

The processing system 600 may be configured to provide the visual representation of the unstructured textual data at the first user interface 620 by controlling the user interface 620 of the system 600.

In some examples, the processing system 600 comprises an output interface 603, which provides output data. The output data may include, for example, a control signal for controlling the operation of the first user interface.

In some embodiments, the present disclosure proposes the use of a machine-learning algorithm to process one or more characteristics of a section to determine a relevance of the section.

A machine-learning algorithm is any self-training algorithm that processes input data in order to produce or predict output data. Here, the input data comprises (values of) one or more characteristics of a section and the output

12

data comprises a relevance of the section.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. $\pm 1\%$) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example (values of) one or more characteristics of a section. The training output data entries correspond to a relevance of the section, e.g. an indicator of relevance.

The training output data entries may be provided by a skilled clinician (or group of skilled clinicians) annotating sample instances of unstructured textual data (which has been divided into section) with manual marks indicating a relevance of different sections. The corresponding example characteristics may be obtained by processing the sample instances according to the processing method that will be implemented.

By way of example, where a user is able to manually indicate a relevance of a section (e.g. by ticking a checkbox alongside the section), then characteristics of this section and the indication that the section is considered relevant may be stored for use in training a machine-learning method.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A computer-implemented method (200, 300) of estimating the relevance of different sections of a medical record of a subject, the computer-implemented method comprising:

    obtaining (210) the medical record of the subject containing unstructured textual data (110);
    dividing (220) the unstructured textual data into a plurality of sections(111, 112, 113, 114, 115, 116) , each section containing a different part of the unstructured textual data;
    providing (230) a visual representation of the unstructured textual data at a first user interface;

visually emphasizing (240) different sections of the unstructured textual data, at the first user interface, responsive to a user input;

monitoring (250), for each section of the unstructured textual data, at least one characteristic of the section of the unstructured textual data, wherein the at least one characteristic comprises a characteristic dependent upon at least one visual emphasization of the section; and

for each section of the unstructured textual data, processing (260) the monitored characteristics to estimate a relevance of the section of the unstructured textual data.

2. The computer-implemented method of claim 1, wherein:

the plurality of sections of the unstructured textual data forms a sequence of sections; and
the step of visually emphasizing different sections of the unstructured textual data comprises visually emphasizing different sections in sequential order, wherein the movement through the sequence is responsive to the user input.

3. The computer-implemented method of any of claims 1 or 2, wherein the at least one characteristic further comprises a user identification of relevance of the section.

4. The computer-implemented method of any of claims 1 to 3, wherein the at least one characteristic comprises a characteristic responsive to a length of time that the section of unstructured textual data is visually emphasized.

5. The computer-implemented method of any of claims 1 to 4, wherein the at least one characteristic comprises a characteristic responsive to a length of time that the section of unstructured textual data is visually emphasized and a measure of size of the section of unstructured textual data.

6. The computer-implemented method of claim 5, wherein the at least one characteristic comprises a characteristic responsive to a measure of size of the section of unstructured textual data divided by the length of time that the section of unstructured textual data is visually emphasized.

7. The computer-implemented method of any of claims 1 to 6, wherein the at least one characteristic comprises a characteristic responsive to a number of times that the user causes the section of unstructured textual data is visually emphasized.

8. The computer-implemented method of any of claims 1 to 7, wherein:

the steps of providing a visual representation of the unstructured textual data and visually emphasizing different sections of the unstructured textual data are repeated for each of a plurality of different users; and
the at least one characteristic comprises a number of different users that cause the section to be visually emphasized.

9. The computer-implemented method of any of claims 1 to 8, wherein:

the step of dividing the unstructured textual data comprises dividing unstructured textual data into a plurality of super-sections, each super-section containing a plurality of sections of unstructured data; and
wherein the at least one characteristic of the section of the unstructured textual data comprises:

a number of times that the super-section in which the section is contained is viewed by the user; and/or.
an identification of whether or not the section is contained in the last super-section viewed by the user before the user ends their interaction with the unstructured medical data.

10. The computer-implemented method of any of claims 1 to 9, wherein the at least one characteristic of the section of the unstructured textual data comprises a proportion of time that the section is visually emphasized with respect to the total time that the user interacts with the medical record or the unstructured medical data.

11. The computer-implemented method of any of claims 1 to 10, wherein the step of visually emphasizing different sections of the unstructured textual data comprises visually emphasizing a section by performing one or more of the following: highlighting the section of unstructured textual data; changing a font size of the section of unstructured textual data; changing a kerning and/or letter-spacing of the section of unstructured textual data; changing a color of the section of unstructured textual data; changing a typographic emphasis of the section of unstructured textual data; moving the section of unstructured textual data and/or text surrounding the section according to a movement pattern; and/or inserting additional space at one or both ends of the section of unstructured textual data.

12. The computer-implemented method (400) of any of claims 1 to 11, further comprising:

    selecting (410) a subset of the sections of the unstructured textual data responsive to estimated relevance of each section, wherein the subset does not include all of the sections of the unstructured textual data; and generating (420) a summarized medical record containing the selected subset of the sections of the unstructured textual data, wherein the summarized medical record does not include all of the sections of the unstructured textual data.

13. The computer-implemented method of claim 12, further comprising a step of displaying the summarized medical record at a second user interface.

14. A computer program product comprising computer program code means which, when executed on a computing device having a processing system, cause the processing system to perform all of the steps of the method according to any of claims 1 to 13.

15. A processing system (50, 600) configured to estimate the relevance of different sections of a medical record, the processing system being configured to:

    obtain (210) the medical record of the subject containing unstructured textual data (110);
    divide (220) the unstructured textual data into a plurality of sections (111, 112, 113, 114, 115, 116), each section containing a different part of the unstructured textual data;
    provide (230) a visual representation of the unstructured textual data at a first user interface;
    visually emphasize (240) different sections of the unstructured textual data, at the first user interface, responsive to a user input;
    monitor (250), for each section of the unstructured textual data, at least one characteristic of the section of the unstructured textual data, wherein the at least one characteristic comprises a characteristic dependent upon at least one visual emphasization of the section; and
    for each section of the unstructured textual data, process (260) the monitored characteristics to estimate a relevance of the section of the unstructured textual data.

100

110    111    112    113

*... Lorem ipsum dolor sit amet, consectetur adipiscing elit,* **sed do eiusmod tempor incididunt ut labore et dolore magna aliqua.** *Ut enim ad minim veniam, quis nostrud exercitation ullamco laboris nisi ut aliquip ex ea commodo consequat. Duis aute irure dolor in reprehenderit in voluptate velit esse cillum dolore eu fugiat nulla pariatur ...*

114

150

115    116

160

FIG. 1

FIG. 2

FIG. 3

```
┌─────────────────────────────┐
│          200, 300           │
└─────────────────────────────┘
               │
               ▼                    410
┌─────────────────────────────┐
│       Select section(s)     │
└─────────────────────────────┘
               │
               ▼                    420
┌─────────────────────────────┐
│   Generate summarized medical│
│            record           │
└─────────────────────────────┘
      430       │        440
  ┌────────────────────┐   ┌────────────────────┐
  │ Store summarized   │   │ Display summarized │
  │  medical record    │   │  medical record    │
  └────────────────────┘   └────────────────────┘

           400
```

FIG. 4

```
┌──────────────────────────────────────────────────┐
│                 53      54      50                 │
│                                                    │
│           ┌──────────────────────────────┐        │
│           │  ┌──────┐      ┌──────┐       │        │
│           │  │      │      │      │       │        │
│   51      │  └──────┘      └──────┘       │        │
│  ┌──────┐ │                               │        │
│  │  μP  │ │                               │        │
│  └──────┘ │    ┌────────────────────┐     │        │
│       55  │    │        O/S         │     │        │
│           │    └────────────────────┘     │        │
│  ┌──────┐ │    ┌────────────────────┐     │        │
│  │ I/O  │ │    │        App         │     │        │
│  └──────┘ │    └────────────────────┘     │        │
│           └──────────────────────────────┘        │
│      57                   56        52             │
└──────────────────────────────────────────────────┘
```

FIG. 5

620　　　600　　　610

Memory　　601　　603　　User Interface

602

60

FIG. 6

Europäisches
Patentamt

European
Patent Office

Office européen
des brevets

# EUROPEAN SEARCH REPORT

**Application Number**

**EP 21 18 7053**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/192143 A1 (KRISHNAN SRIRAM [US] ET AL) 16 August 2007 (2007-08-16) * paragraph [0023] – paragraph [0146] * * figure 6 * ----- | 1-15 | INV. G16H10/60 |
| A | WO 2011/132097 A2 (KONINKL PHILIPS ELECTRONICS NV [NL]; QIAN YUECHEN [US] ET AL.) 27 October 2011 (2011-10-27) * page 6, line 4 – page 22, line 25 * ----- | 1-15 | |

**TECHNICAL FIELDS SEARCHED (IPC)**

G16H

The present search report has been drawn up for all claims

| Place of search | Date of completion of the search | Examiner |
|---|---|---|
| Munich | 14 December 2021 | Rinelli, Pietro |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 18 7053

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2021

| Patent document cited in search report | Publication date | Patent family member(s) | Publication date |
|---|---|---|---|
| US 2007192143 A1 | 16-08-2007 | NONE | |
| WO 2011132097 A2 | 27-10-2011 | BR 112012026477 A2 | 09-08-2016 |
| | | CN 102844761 A | 26-12-2012 |
| | | EP 2561458 A2 | 27-02-2013 |
| | | JP 5744182 B2 | 01-07-2015 |
| | | JP 2013525898 A | 20-06-2013 |
| | | US 2014149407 A1 | 29-05-2014 |
| | | WO 2011132097 A2 | 27-10-2011 |

For more details about this annex : see Official Journal of the European Patent Office, No. 12/82